# EUROPEAN PATENT SPECIFICATION

(11) **EP 1 813 216 B1**
(45) Date of publication and mention of the grant of the patent: **07.10.2015**
(21) Application number: 07100963.3
(22) Date of filing: 23.01.2007
(51) Int. Cl.: A61B 17/86

(54) **Screw for performing syndesis of a fracture**
Schraube für die Syndese einer Fraktur
Vis pour réaliser l'accolement d'une fracture

(30) Priority: 27.01.2006 IT BO20060009 U
(43) Date of publication of application: 01.08.2007
(73) Proprietor: CITIEFFE S.r.l., 40012 Calderara di Reno (Bologna) (IT)
(72) Inventor: Mingozzi, Franco, 40012, Calderara di Reno BO (IT); Dovesi, Alan, 40138, Bologna (IT); Caiaffa, Vincenzo, 70124, Bari (IT)
(74) Representative: Modiano, Micaela Nadia

(56) References cited:
- EP-A- 1 240 874
- EP-A2- 0 263 938
- WO-A1-96/13219
- DE-U1- 20 014 648
- US-A1- 2002 087 161
- US-A1- 2005 277 940

## Description

The present invention relates to a screw for the syndesis of fractures. The invention is aimed at proposing a screw for reducing fractures in general and particularly but not exclusively fractures of the trochanter and of the neck of the femur.

Fractures of the trochanter and of the neck of the femur are reduced by using screws (also known as head screws) constituted by an elongated stem in which one end, after passing through the trochanter, is screwed into the head of the femur. Since said screws are driven through a hole which is obliquely inclined with respect to the axis of the femur, the head of the screw consequently rests on the trochanter cortex only with a small angular portion of its perimetric rim, so that once installed it assumes an inclined arrangement which can lead to localized failures of the cortex and to a loosening of the clamping force applied to the fracture region.

DE 200 14 648 discloses a condylus bone screw having a combination of features as set forth in the pre-characterizing portion of the appended claim 1.

The aim of the present invention is to provide a screw which allows to avoid the above-mentioned drawbacks.

Within this aim, an object of the present invention is to provide a screw which can be implanted with high precision with the aid of a guiding wire.

In accordance with the invention, there is provided a screw for syndesis of a fracture, as defined in the appended claim 1. Preferred embodiments are defined in the dependent claims.

Further characteristics and advantages of the screw according to the invention will become better apparent from the following detailed description of an embodiment thereof, illustrated only by way of nonlimiting example in the accompanying drawings, wherein:
Figure 1 is a perspective view of the screw.
Figure 2 is a side view of the screw of Figure 1;
Figure 3 is a sectional view, taken along the line III-III of Figure 2;
Figure 4 is an enlarged-scale view of the detail enclosed in the circle of Figure 3;
Figure 5 is a view of a fractured femur after reduction of the fracture by means of a pair of screws.

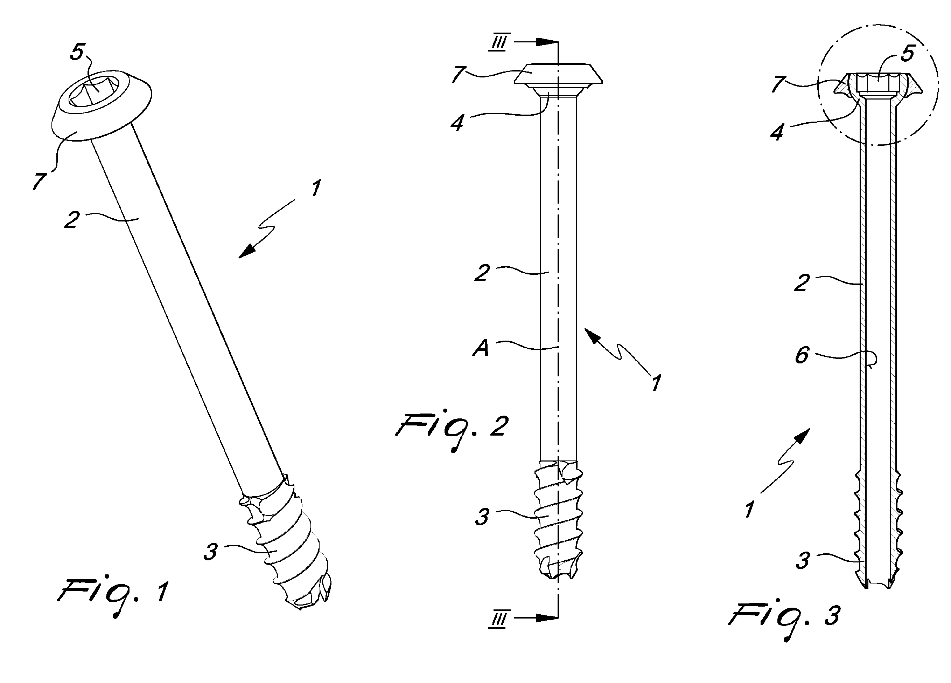

With reference to Figures 1 to 4, a screw according to the invention is generally designated by the reference numeral 1 and comprises a substantially cylindrical stem 2 which has an axis A and has, at one end, a threaded portion 3 and, at the opposite end, a head 4 which is substantially hemispherical and has a larger diameter than the stem.

A recess 5 is formed in the head 4 and has a hexagonal cross-section in order to receive a screw driving tool, specifically an Allen key.

The stem 2 is crossed axially by a channel 6 which leads into the recess 5, so that the stem assumes a tubular structure.

A ring 7 is mated with the head 4 and forms a flange for the abutment of the screw on the cortex of the bone through which the screw 1 is to be implanted.

In particular, the ring 7 has a substantially triangular cross-section, with an internal face which integrates with an annular lip 8, forming a concave internal surface 9 which is spherically complementary to the surface of the head 4. In this manner, the ring 7 and the head 4 form a spherical coupling which allows the ring 7 to assume an inclined position with respect to the axis A until the annular lip 8 engages in the groove 10 formed by the head 4 and by the stem 2. Conveniently, coupling is provided by inserting by interference the head 4 in the cavity formed by the concave surface 9 so that the ring 7 is retained articulately on the head.

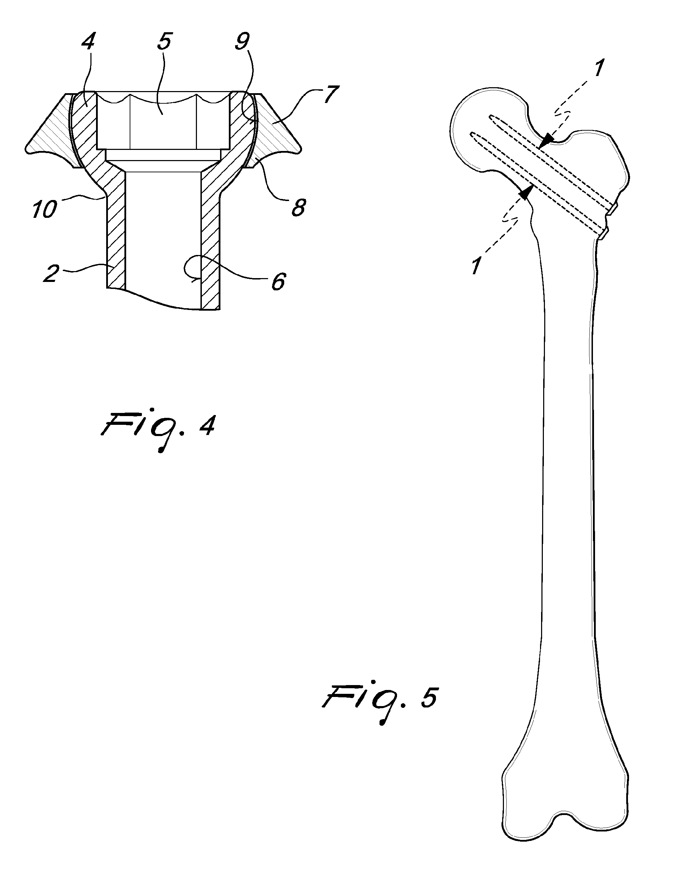

The method of installing the screw 1 should be fully evident from the above and can be also deduced from Figure 5, which illustrates merely by way of example the way in which the syndesis screw works in the case of a fracture of the head of the femur.

After setting the fracture (the plane of which, as usually occurs in femoral fractures, is inclined with respect to the axis of the femur), a hole is provided which, through the plane of the fracture, extends into the head of the femur and allows the insertion of a wire (known in the field as Kirschner's wire), which is designed to guide the implantation of the screw. Then, after positioning the screw by passing it along the wire, said screw is implanted by means of the screwdriver tool, which is engaged in the recess 5 and is turned until the ring 7 abuts against the cortex and the intended clamping of the fractured parts is achieved.

It is significantly important that the ring 7 remains perfectly rested against the femoral surface, so as to avoid edge contact and thus load stresses in localized regions which might lead to failures of the bone and compromise the clamping force.

The described invention therefore achieves the intended aim and objects. In particular, it is noted that in the case of very tough bones the implantation of the screw can be preceded by a widening of the implantation hole by using a tubular reamer which is guided on the same wire as the screw.

It is obvious that the provided number of screws to be implanted is chosen as a function of the extension and orientation of the fracture. In the example shown in Figure 5, in order to ensure effective syndesis of the head of the femur, two mutually parallel screws are used. Likewise, the length and diameter of the screws are chosen in relation to the dimensions of the bone parts in which they are to be implanted.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A screw for syndesis of a fracture, comprising a tubular stem (2) which is configured to be inserted in a hole which passes through the fracture region and has, at one end, a threaded portion (3) and, at the opposite end, a substantially hemispherical head (4), and a groove (10) formed between said head (4) and said stem (2), an abutment ring (7) being arranged on said stem (2) and being mated with said head (4) and having a concave internal surface (9) which is complementary to the hemispherical surface of said head (4) so as to provide a spherical coupling which is suitable to allow said ring (7) to rest flat on the region of the cortex that surrounds the hole in which said stem (2) is inserted, **characterized in that** said ring (7) has a triangular cross-section, the ring (7) having a maximum outer diameter bigger than the outer diameter of the proximal end of the ring (7), wherein a distal lower surface of the ring (7) for abutting on the cortex is formed by a non-planar concave curved lower surface starting from said maximum diameter and extending downwardly towards the stem (2), said ring further comprising an annular lip (8) formed by the inner portion of said non-planar concave curved lower surface and the distal lower portion of the concave internal surface (9) and which extends toward the goove (10).

2. The screw according to claim 1, **characterized in that** said head (4) has a recess (5) which is suitable to receive a tool for tightening the screw (1) and is connected to the channel (6) which passes through said tubular stem (2).

3. The screw according to one of claims 1 to 2, **characterized in that** said spherical coupling is provided by inserting with interference the head (4) in the spherical cavity formed by said concave surface (9) so that the ring (7) is retained articulately on the head (4).

## Patentansprüche

1. Schraube fur die Syndese einer Fraktur, mit einem rohrförmigen Schaft (2), der ausgestaltet ist, um in ein Loch eingefuhrt zu werden, das durch den Frakturbereich verlauft, und an einem Ende einen Gewindeabschnitt (3) und an dem gegenuberliegenden Ende einen im Wesentlichen halbkugeligen Kopf (4) sowie eine zwischen dem Kopf (4) und dem Schaft (2) ausgebildete Auskehlung (10) aufweist, wobei ein Anschlagring (7) auf dem Schaft (2) angeordnet und mit dem Kopf (4) gepaart ist und eine konkave innere Flache (9) hat, die komplementar zu der halbkugeligen Flache des Kopfs (4) ausgebildet ist, um ein Kugelgelenk zu schaffen, das geeignet ist, es dem Ring (7) zu ermöglichen, flach auf dem Bereich des Kortex aufzuliegen, der das Loch umgibt, in dem der Schaft (2) eingesetzt ist, **dadurch gekennzeichnet, dass** der Ring (7) einen dreieckigen Querschnitt hat, wobei der Ring (7) einen maximalen AuBendurchmesser aufweist, der grower ist als der Außendurchmesser des proximalen Endes des Rings (7), und wobei eine distal untere Fläche des Rings (7) zum Auflegen auf den Kortex durch eine nicht-ebene, konkav gekrümmte, untere Fläche geformt ist, die am maximalen Durchmesser beginnt und sich nach unten in Richtung des Schafts (2) erstreckt, und wobei der Ring ferner eine ringförmige Lippe (8) aufweist, die durch den inneren Abschnitt der nicht-ebenen, konkav gekrümmten, unteren Fläche und den distal unteren Abschnitt der konkaven inneren Flache (9) gebildet ist und welche sich in Richtung der Auskehlung (10) erstreckt.

2. Schraube nach Anspruch 1, **dadurch gekennzeichnet, dass** der Kopf (4) eine Aussparung (5) aufweist, die dazu geeignet ist, ein Werkzeug zum Festziehen der Schraube (1) aufzunehmen, und die mit dem Kanal (6) verbunden ist, der durch den rohrförmigen Schaft (2) verläuft.

3. Schraube nach einem der Ansprüche 1 bis 2, **dadurch gekennzeichnet, dass** das Kugelgelenk durch Einsetzen des Kopfs (4) in die kugelige Aushöhlung geschaffen ist, die durch die konkave Fläche (9) mit Hinterschnitt gebildet ist, so dass der Ring (7) gelenkig am Kopf (4) gehalten ist.

## Revendications

1. Vis pour réaliser l'accolement d'une fracture, comprenant une tige tubulaire (2) qui est configurée pour être insérée dans un trou qui passe à travers la région de la fracture et qui a, à une extrémité, une portion filetée (3) et, à l'extrémité opposée, une tête sensiblement hémisphérique (4), et une rainure (10) formée entre ladite tête (4) et ladite tige (2), une bague de butée (7) étant agencée sur ladite tige (2) et étant accouplée avec ladite tête (4) et ayant une surface interne concave (9) qui est complémentaire de la surface hémisphérique de ladite tête (4) de manière à réaliser un couplage sphérique qui convient pour permettre à ladite bague (7) de reposer à plat sur la région du cortex qui entoure le trou dans lequel ladite tige (2) est insérée, **caractérisée en ce que** ladite bague (7) a une section transversale triangulaire, la bague (7) ayant un diamètre extérieur maximum plus grand que le diamètre extérieur de l'extrémité proximale de la bague (7), dans laquelle une surface inférieure distale de la bague (7) destinée à venir buter sur le cortex est formée par une surface inférieure incurvée concave non planaire qui commence depuis ledit diamètre maximum et qui s'étend vers le bas en direction de la tige (2), ladite bague comprenant en outre une lèvre annulaire (8) formée par la portion intérieure de ladite surface inférieure incurvée concave non planaire et par la portion inférieure distale de la surface interne concave (9) et qui s'étend vers la rainure (10).

2. Vis selon la revendication 1, **caractérisée en ce que** ladite tête (4) a un évidement (5) qui est approprié pour recevoir un outil pour serrer la vis (1) et qui est connecté au canal (6) qui passe à travers ladite tige tubulaire (2).

3. Vis selon l'une des revendications 1 et 2, **caractérisée en ce que** ledit couplage sphérique est assuré en insérant avec interférence la tête (4) dans la cavité sphérique formée par ladite surface concave (9) de sorte que la bague (7) est retenue de manière articulée sur la tête (4).
